# EUROPEAN PATENT SPECIFICATION

(11) **EP 0 432 303 B1**
(45) Date of publication and mention of the grant of the patent: **13.04.1994**
(21) Application number: 89123136.7
(22) Date of filing: 14.12.1989
(51) Int. Cl.: A61F 5/01

(54) **Cuff device**
Orthese
Prothèse d'articulation

(43) Date of publication of application: 19.06.1991
(73) Proprietor: Spademan, Richard G., Sacramento California 95816 (US)
(72) Inventor: Spademan, Richard G., Sacramento California 95816 (US)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) References cited:
- GB-A- 2 139 896
- US-A- 2 467 907
- US-A- 4 506 661
- US-A- 4 856 500

## Description

### BACKGROUND OF THE INVENTION

The present invention relates to therapeutic and prophylactic devices, particularly to a cuff device or dynamic support that temporarily tightens and loosens on a wearer's body part as another body part is moved.

Various compressive cuff devices are known such as the straps that hold braces on a patient's limb and trunk to protect ligaments, tendons and bones as they heal following injury or surgery. Various strapping devices are also used to help prevent injury or provide support for the chronic instability of a body part. Elastic stockings and inflatable cuffs are used to reduce edema and blood stasis in the extremities that result from disease, injury, prolonged confinement or surgery.

Unfortunately, at the present time, ideal conditions for the efficient application of these braces, cuffs and stockings cannot be achieved with conventional means. These supporting structures tend to be either too loose on the body part, in which case the support members cannot adequately stabilize the body part or, more frequently, these supporting structures are held too tightly, intensifying discomfort, prolonging immobility and aggravating the problem of stasis or atrophy.

US-A-4 856 500 teaches a dynamic support for first and second body parts which are articulated to each other comprising cuff components adapted to engage the first and second body parts when the first and second body parts in a resting position, the cuff components engaging the first and second body parts at locations spaced from the body area where the body parts are articulated, arms attached to and extending from each of the cuff components and terminating in end regions, said end regions being movable in attachment to each other at a point adjacent to the area where the body parts are articulated, a tightening system attached to the cuff components and including members responsive to a predetermined relative movement between the body parts in more than one direction away from the resting position for increasing the tightness with which the cuff components engage the body parts.

The purpose of this known dynamic support is however to dynamically and temporarily tighten the fitting system differentially and progressively on the body parts in response to movement of one body part relative to the other body part, or to provide a dynamic support that momentarily tightens on a body part in response to movement of another body part.

### SUMMARY OF THE INVENTION

It is an object of the present invention to provide dynamic supports that can temporarily tighten and loosen from a close fit or snug position on the body parts and balance the tightness of the supports in response to a predetermined movement in a predetermined direction from a predetermined position of one of the body parts.

### BRIEF DESCRIPTION OF THE DRAWINGS

In the drawings:
Fig. 1 is a front elevation view of the dynamic support in an extended position.
Fig. 2 is a back elevation view of the dynamic support in an extended position.
Fig. 3 is a side elevation view of the dynamic support in a flexed position.
Fig. 4 is a side elevation view of the dynamic support in an extended position.
Fig. 5 is a vertical sectional view taken along line A-A of Fig. 4.
Fig. 6 is a front elevation view of the dynamic support of an alternative embodiment of the present invention.
Fig. 7 is a side elevation view of the dynamic support of the alternative embodiment of the present invention showing the pivot and cable assembly pivoted from the resting position.
Fig. 8 is a vertical sectional view taken along line B-B of Fig. 7.

### DESCRIPTION OF THE PREFERRED EMBODIMENTS

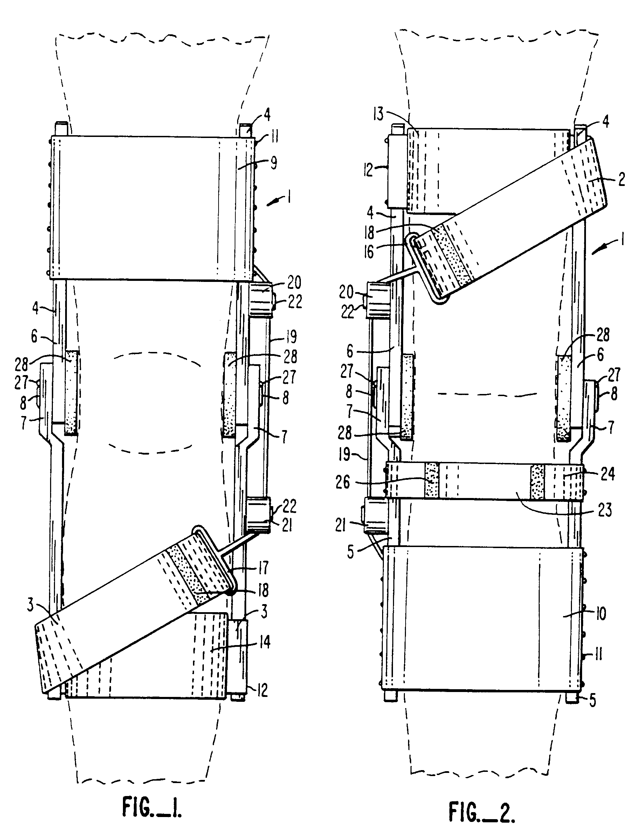

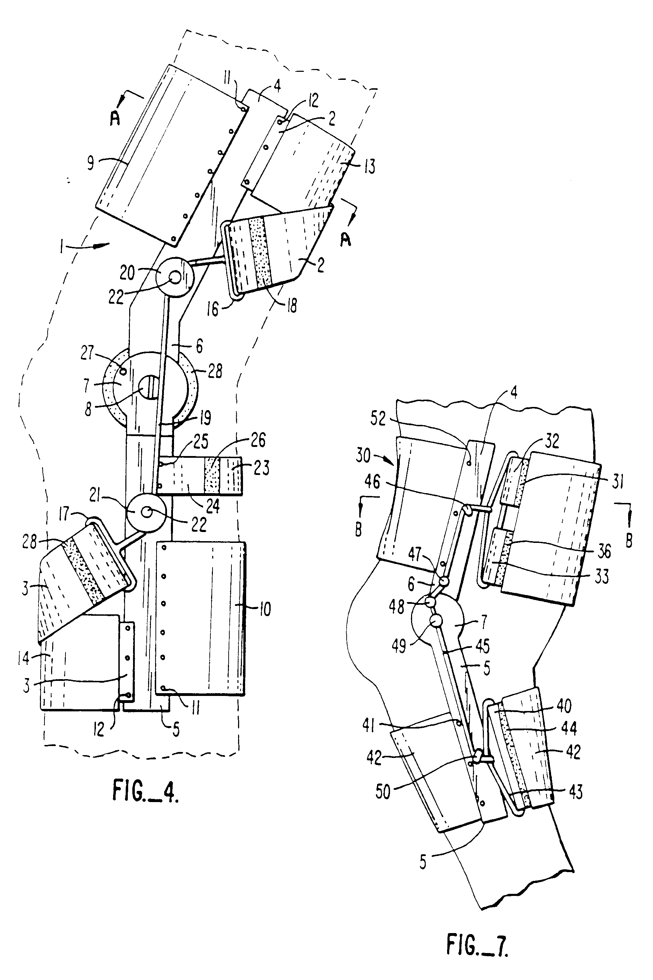

Referring to Figs. 1-5, a cuff device or dynamic support for the lower extremity and particularly the knee, is shown, but it is understood that the principles of the invention are also applicable to other articulated body parts. There is shown in Fig. 1 a dynamic support 1 which includes an upper or first body part engaging cuff component 2 and a lower or second body part engaging cuff component 3. These cuff components comprise a fitting system and are respectively adapted to engage the body parts above and below the joint.

A pair of arms 4 and 5 are respectively attached to and extend toward each other from the body part engaging cuff components 2 and 3. A set of these arms are located on opposite sides of the extremity. Each of these arms 4 and 5 terminate in movable overlapping end regions 6 and 7 remote from the cuff components and are formed with aligned openings through which a single pivot pin 8 extends. A more complex slidable and pivotable orthotic joint can be used. The orthotic joint can incorporate a rivet limit stop 27 known per se. The pivotable orthotic joint may also incorporate a condylar pad 28 known per se secured to the pivot pin 8. End region 7 pivots freely about pivot pin 8. Thus, pivot pin 8 forms a pivot axis which is generally perpendicular to the arms 4 and 5 and which coincides with the predominant axis to which swinging of the upper and lower body parts are limited. The arms 4 and 5 can be of a slightly flexible material construction such as metal or plastic. There is an anterior thigh upper cross member 9 and posterior calf lower cross member 10 which can be made of a slightly flexible formable material such as metal or plastic as are the arms 4 and 5 and can be shaped to conform to the body part such as the thigh and leg with heat and shaping tools. The opposite ends of each of the cross members 9 and 10 are fixed to the ends of each set of the arms 4 and 5 respectively by rivets 11 or the like.

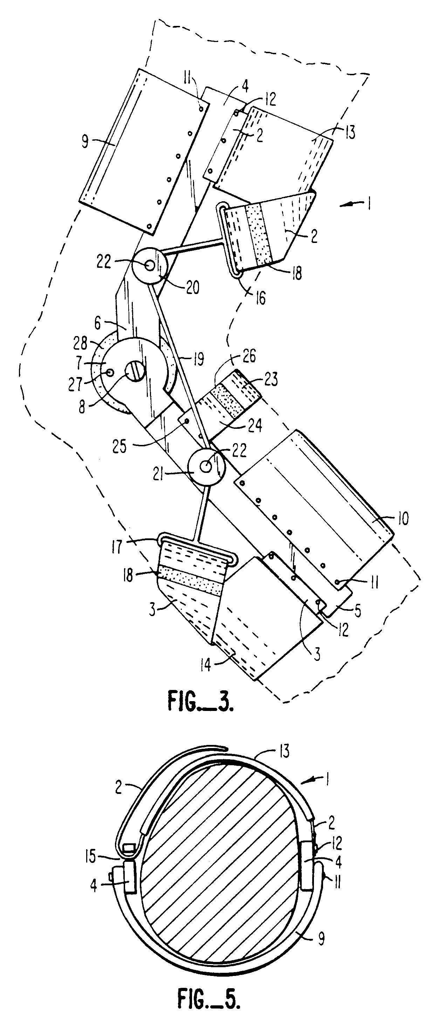

The upper cuff component 2 and the lower cuff component 3 are constructed of slightly yieldable material such as plastic or cloth to conform to the body part configuration. Each of the cuff components 2 and 3 are attached at one end to one of the arms 4 and 5 respectively by rivets 12 or the like. The cuff components 2 and 3 pass through sleeves 13 and 14 respectively which are constructed of neoprene materials or the like, pass through a slot 15 located toward the end of one of the uprights 4 and 5, as shown in Fig. 3, and are adjustable and releasable in fixation to themselves by a Velcro (R) strip 18 or the like. The loops 16 and 17 are attached to a cable 19 which passes through guide 20 and guide 21 attached to one set of the arms 4 and 5 respectively by a bolt 22 or the like. Straps 23 and 24 are each attached at one end to one of the opposite arms 5 by rivets 25 or the like and are adjustable and releasable in fixation to each other by a Velcro (R) strip 26 or the like. Straps 23 and 24 resist forward migration on the knee of end regions 6 and 7.

Movement of hinging end regions 6 and 7 causes the cable 19 to tighten cuff components 2 and 3 on the extremity by relative shortening of the cable 19 when the thigh is moved to an extended position relative to the leg from the flexed position. The segment of the upper cuff component 2 and lower cuff component 3 that pass through the sleeves 13 and 14 respectively tighten generally transverse the long axis of the body part. The segment of the upper cuff component 2 and the segment of the lower cuff component 3 that do not pass through the sleeves 13 or 14 respectively also tighten with a torsional component in generally opposite directions on the first and second body parts. Since the upper and lower cuff components are connected by the cable 19 there will be a balancing of the amount of tightening of the upper and lower cuff components.

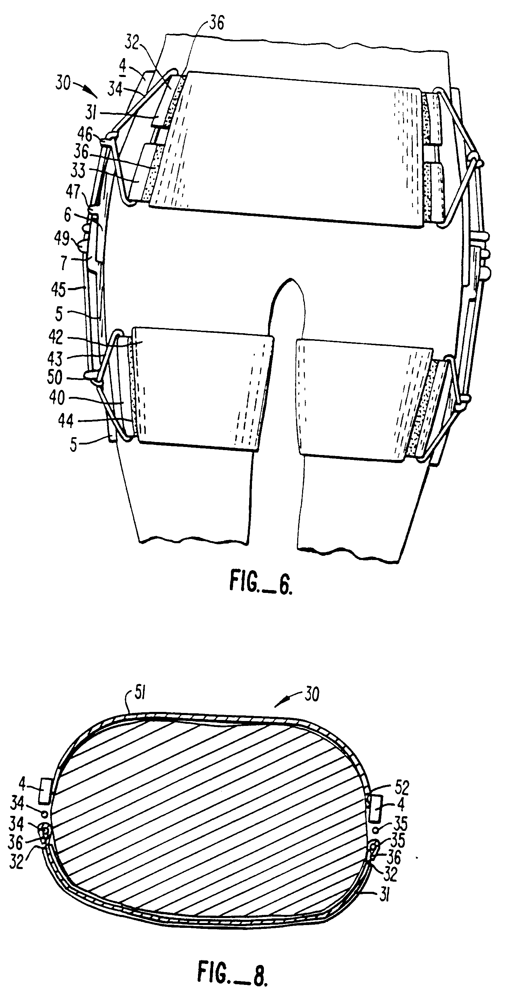

Referring to Figs. 6-8, there is provided in accordance with another embodiment of the present invention, a dynamic support designated generally as 30. Except as hereinafter described, dynamic support 30 is substantially identical to the dynamic support 1 and operates in the same manner with a tightening system for dynamically and momentarily tightening the cuff components on the body parts from the snug close fit position. Those features of the dynamic support which are identical to those of the dynamic support of Figs. 1-5 are identified using the same numbers used in each side of the body. Arms 4 and 5 and end regions 6 and 7 and pivot pin 8 operate in the same manner as those parts shown in Figs. 1-5.

An upper or first body part engaging cuff component 31 can be constructed of slightly yieldable material such as plastic or cloth and consists of a plurality of straps 32 and 33 which pass, on each end, through a loop 34 on each side of the first body part or trunk. Each strap 32 and 33 is adjustable and releasable in fixation to itself by a Velcro ^{R} strip 36 or the like. A lower or second body part engaging cuff component 40 constructed of slightly yieldable material such as plastic or cloth is attached at one end to lower arm 5 by rivets 41 or the like. The lower cuff component 40 passes through a sleeve 42, around the second body part or lower thigh and through a loop 43 and is adjustable and releasable in fixation to itself by a Velcro ^{R} strip 44 or the like. The loops 34 and 43 are attached to a cable 45, which passes through guide 46 and guide 47 which is attached by a bolt in a slot 48 in upper arm 4, guide 49 on end region 7 and guide 50 on lower arm 5. The rate and amount of tightness of the upper and lower cuff components or dynamic supports can be adjusted by the position of guide 47 in slot 48.

There is a posteriorly located cross member 51 which can be made of slightly flexible formable material such as metal or plastic and can be shaped to conform to the body part, such as the trunk, with heat and shaping tools. The opposite ends of the cross member 51 are attached to the ends of each arm 4 by rivets 52 or the like.

In use, movement of hinging end regions 6 and 7 cause the cable 44 to tighten cuff components 31 and 32 on the trunk and thigh when the trunk and thigh are moved in flexion or extension from the resting position by the relative shortening of cable 45 as end region 7 pivots relative to end region 6 increasing the distance between guide 47 and guide 48.

Details have been disclosed to illustrate the invention in a preferred embodiment of which adaptation and modification within the scope of the invention as defined by the appended claims will occur to those skilled in the art. For instance, the pivot and cable assembly can be incorporated into a dynamic support or cuff device to tighten and loosen from the close fit resting position on various body parts such as the trunk and shoulder, arm and forearm and wrist and hand.

## Claims

1. A dynamic support (1) for first and second body parts which are articulated to each other, comprising cuff components (2, 3) adapted to engage the first and second body parts when the first and second body parts are in a resting position, the cuff components (2, 3) engaging the first and second body parts at locations spaced from the body area where the body parts are articulated, arms (4, 5) attached to and extending from each of the cuff components (2, 3) and terminating in end regions (6, 7), said end regions being movable in attachment to each other at a point adjacent to the area where the body parts are articulated, a tightening system attached to the cuff components and including members (16, 17, 18, 19, 20, 21, 22) responsive to a predetermined relative movement between the body parts in more than one direction away from the resting position for increasing the tightness with which the cuff components engage the body parts,
**characterized** in that
said members (16, 17, 18, 19, 20, 21, 22) include means (19) for balancing the tightness with which the cuff components (2, 3) engage the first and second body parts.

2. A dynamic support according to claim 1,
**characterized** in that
said tightening system includes at least one movable member (19) connecting the cuff components.

3. A dynamic support according to claim 2,
**characterized** in that
said connecting member comprises a cable (19).

4. A dynamic support according to claim 3,
**characterized** in that
each said cuff component comprises a loop (16, 17), said cable (19) being attached at its ends to a respective loop and passing through guides (20, 21) attached to the arms (4, 5).

5. A dynamic support according to anyone of claims 1 to 4,
**characterized** in that
said tightening means impart a torsional component to said cuff members (2, 3).

6. A dynamic support according to claim 5,
**characterized** in that
said torsional component tightens in substantially opposite directions on said first and second body parts.

7. A dynamic support according to anyone of claims 1 to 6,
**characterized** in that
said tightening system includes members (47, 48) for adjusting the rate and amount of tightness with which the cuff components (2, 3) engage the first and second body parts.

## Patentansprüche

1. Ein dynamischer Träger (1) für erste und zweite Körperteile, welche gelenkig miteinander verbunden sind, mit Manschettenkomponenten (2, 3), die dazu ausgebildet sind, mit den ersten und zweiten Körperteilen in Eingriff zu kommen, wenn sich die ersten und zweiten Körperteile in einer Ruheposition befinden, wobei die Manschettenkomponenten (2, 3) mit den ersten und zweiten Körperteilen an Orten in Eingriff treten, die von der Körperfläche beabstandet sind, wo die Körperteile gelenkig miteinander verbunden sind,
Schenkeln (4, 5), die an den Manschettenkomponenten (2, 3) angebracht sind und sich von diesen erstrecken und in Endbereichen (6, 7) enden, wobei die Endbereiche in der Anbringung aneinander an einem Punkt benachbart der Fläche, wo die Körperteile gelenkig miteinander verbunden sind, bewegbar sind, einem Straffungssystem, das an die Manschettenkomponenten angebracht ist und Glieder (16, 17, 18, 19, 20, 21, 22) umfaßt, die auf eine vorbestimmte relative Bewegung zwischen den Körperteilen in mehr als einer Richtung weg aus der Ruheposition zur Erhöhung der Straffheit ansprechen, mit welcher die Manschettenkomponenten mit den Körperteilen in Eingriff treten,
dadurch **gekennzeichnet,**
daß die Glieder (16, 17, 18, 19, 20, 21, 22) ein Mittel (19) zum Ausgleichen der Straffheit umfassen, mit welcher die Manschettenkomponenten (2, 3) mit den ersten und zweiten Körperteilen in Eingriff treten.

2. Ein dynamischer Träger nach Anspruch 1,
dadurch **gekennzeichnet,**
daß das Straffungssystem zumindest ein bewegbares Glied (19) umfaßt, das die Manschettenkomponenten verbindet.

3. Ein dynamischer Träger nach Anspruch 2,
dadurch **gekennzeichnet,**
daß das Verbindungsglied ein Kabel (19) umfaßt.

4. Ein dynamischer Träger nach Anspruch 3,
dadurch **gekennzeichnet,**
daß jede Manschettenkomponente eine Schleife (16, 17) umfaßt, wobei das Kabel (19) an seinen Enden an einer jeweiligen Schleife angebracht ist und durch Führungen (20, 21) hindurch tritt, die an den Schenkeln (4, 5) angebracht sind.

5. Ein dynamischer Träger nach einem der Ansprüche 1 bis 4,
dadurch **gekennzeichnet,**
daß das Straffungsmittel den Manschettengliedern (2, 3) eine Torsionskomponente verleiht.

6. Ein dynamischer Träger nach Anspruch 5,
dadurch **gekennzeichnet,**
daß die Torsionskomponente in im wesentlichen entgegengesetzten Richtungen eine Straffwirkung auf den ersten und zweiten Körperteilen ausübt.

7. Ein dynamischer Träger nach einem der Ansprüche 1 bis 6,
dadurch **gekennzeichnet,**
daß das Straffungssystem Glieder (47, 48) zum Einstellen der Rate und des Betrages der Straffheit umfaßt, mit welcher die Manschettenkomponenten (2, 3) mit den ersten und zweiten Körperteilen in Eingriff treten.

## Revendications

1. Support dynamique (1) pour une première et une seconde parties de corps qui sont articulées l'une à l'autre, comprenant des composants en forme de manchette (2, 3) adaptés pour s'appliquer étroitement sur les première et seconde parties de corps lorsque ces dernières sont dans une position de repos, les composants en forme de manchette (2, 3) engageant les première et seconde parties de corps à des emplacements éloignés de la zone du corps où les parties de corps sont articulées, des bras (4, 5) attachés à chacun des composants en forme de manchette (2, 3), s étendant depuis lesdits composants et se terminant par des régions d'extrémité (6, 7), lesdites régions d'extrémité étant attachées de façon mobile l'une à l'autre en un point voisin de la zone où les parties de corps sont articulées, un système de tensionnement étant relié aux composants en forme de manchette et comprenant des organes (16, 17, 18, 19, 20, 21, 22) qui réagissent à un mouvement relatif prédéterminé entre les parties de corps dans plus d'une direction en éloignement de la position de repos pour augmenter le serrage sous lequel les composants en forme de manchette (2, 3) engagent les parties de corps,
caractérisé en ce que lesdits organes (16, 17, 18, 19, 20, 21, 22) comprennent des moyens (19) pour équilibrer le serrage sous lequel les composants en forme de manchette (2, 3) engagent les première et seconde parties de corps.

2. Support dynamique selon la revendication 1, caractérisé en ce que ledit système de tensionnement comprend au moins un organe mobile (19) qui relie les composants en forme de manchette.

3. Support dynamique selon la revendication 2, caractérisé en ce que ledit organe de liaison comprend un câble (19).

4. Support dynamique selon la revendication 3, caractérisé en ce que chacun desdits composants en forme de manchette comprend une boucle (16, 17), ledit câble (19) étant attaché à ses extrémités à une boucle respective et passant à travers des guides (20, 21) attachés aux bras (4, 5).

5. Support dynamique selon l'une quelconque des revendications 1 à 4, caractérisé en ce que lesdits moyens de tensionnement imposent une composante de torsion auxdits composants en forme de manchette (2, 3).

6. Support dynamique selon la revendication 5, caractérisé en ce que ladite composante de torsion effectue un serrage dans des directions sensiblement opposées sur lesdites première et seconde parties de corps.

7. Support dynamique selon l'une quelconque des revendications 1 à 6, caractérisé en ce que ledit système de tensionnement comprend des organes (47, 48) pour ajuster le taux et l'intensité du serrage sous lequel les composants en forme de manchette (2, 3) engagent les première et seconde parties de corps.
